# EUROPEAN PATENT APPLICATION

(11) **EP 2 952 931 A1**
(43) Date of publication of application: **09.12.2015**
(21) Application number: 15170376.6
(22) Date of filing: 03.06.2015
(51) Int. Cl.: G01T 1/16, G01R 33/48

(54) **MRI-PET CEPHALIC MOLECULAR IMAGING COIL AND MRI-PET CEPHALIC MOLECULAR IMAGING SYSTEM**

(30) Priority: 05.06.2014 CN 201410244147
(71) Applicant: Beijing Top Grade- Kang Ming Medical Device Inc., Beijing 100176 (CN)
(72) Inventor: Sun, Keke, Beijing Beijing 100176 (CN); Sun, Ming, Beijing Beijing 100176 (CN)
(74) Representative: Platzöder, Michael Christian

(57) **Abstract**

This invention discloses an MRI-PET cephalic molecular imaging coil (10) and an MRI cephalic molecular imaging system including this MRI-PET cephalic molecular imaging coil (10); the said MRI-PET cephalic molecular imaging coil (10) is an integrated and nested structure, with a PET molecular signal acquisition assembly (12) installed on the peripheral surface of the MRI cephalic RF coil (11) , the said PET molecular signal acquisition assembly (10) including a crystal gamma-ray detector (15) and photoelectric conversion structure (16); the said photoelectric conversion structure (16) is semi-conductors, having an effect in preventing the impact of magnetic field on gamma-ray imaging, hence making it possible to obtain precision fusion images of magnetic resonance and molecular imagery and ensuring consistency of physiological and biochemical information in spatial location and temporal synchronization.

## Description

### Technical Field of Invention

The present invention relates to the field of nuclear magnetic resonance analysis and nuclear medical imaging, and more particularly, to an MRI-PET cephalic molecular imaging coil and a system including the said MRI-PET cephalic molecular imaging coil.

### Technical Background

Given the extensive application of nuclear magnetic resonance imaging (MRI) in basic research such as chemistry and biology and also in clinic medicine and pharmaceutical R&D, many kinds of high resolution nuclear magnetic resonance analysis techniques have become an important tool in biochemistry research. With the rapid development of transmission imaging on the structurology and anatomy, the physiological and biochemical information imaging also becomes an important component. At present, the more advanced technology of fusion-imaging with information of anatomy structure and physiological biochemistry has been developed in field of science and clinic, this technology has improved our understanding of diseases from cellular dysfunction to pathological changes, and has provided more information for clinic studies and scientific research.

On the other hand, the research is still making progress on the imaging fusion of MRI and PET. The common method is that the image fusion was taken place after separated acquisition of molecular image equipment such as PET (positron emission computed tomography) and MRI. The method has many disadvantages, however, such as huge volume and expensive price. In addition, the limitation of the current technology is the problem of acquisition accuracy, which is caused by has separated scanning of PET and MRI. The data, which was not generated simultaneously from PET and MRI, has caused certain deviation in fusion-images. That is the fusion information was not come from synchronous data acquisition. By contrast, the fusion-images could be more accurate if those imaged are generated at same space, at same time, and with same metabolic situations.

### Detailed Description of the Present Invention

Aiming at the deficiency existing in the prior art, the present invention has as its objective to provide an MRI-PET cephalic molecular imaging coil and a system including the MRI-PET cephalic molecular imaging coil. The MRI-PET cephalic molecular imaging system improves on current nuclear magnetic cephalic coil and uses a new type of coil to achieve molecular image acquisition. In addition to such benefits as compact equipment, simple companion hardware, and convenient use, the present invention is able to generate precision fusion images of magnetic resonance image and molecular image (molecular MR image) and ensure biochemical information consistency in spatial location and temporal synchronization.

To achieve the above objective, on the one hand, the present invention provides an MRI-PET cephalic molecular imaging coil, including traditional MRI cephalic RF coil and PET molecular signal acquisition assembly, the MRI-PET cephalic molecular imaging coil is an integrated and nested structure wherein the PET molecular signal acquisition assembly is located on the peripheral surface of the MRI cephalic RF coil; the molecular signal acquisition assembly includes a crystal gamma-ray detector and a photoelectric conversion structure, the photoelectric conversion structure is semi-conductors, effective in preventing the impact of magnetic field on gamma-ray imaging. Preferably, the PET molecular signal acquisition assembly is attached to the peripheral surface of the MRI cephalic RF coil so as to form an ring shape.

Preferably, the PET molecular signal acquisition assembly is attached to the peripheral surface of the MRI cephalic RF coil and the regions of the MRI cephalic RF coil not attached to the PET molecular signal acquisition assembly are provided with windows.

Preferably, the PET molecular signal acquisition assembly includes multiple unit structures, each unit structure is composed of a crystal gamma-ray detector unit and a photoelectric conversion structure unit.

Preferably, the crystal gamma-ray detector is attached to the peripheral surface of said MRI cephalic RF coil, and the photoelectrical conversion structure is attached to the peripheral surface of the crystal gamma-ray detector.

Preferably, the ray crystal detector is selected from BGO, LYSO, LSO, or LBS gamma-ray detection crystals.

On the other hand, the present invention also provides an MRI-PET cephalic molecular imaging system, which includes the MRI-PET cephalic molecular imaging coil, signal processing assembly, and reconstruction and fusion workstation, wherein the signal processing assembly is connected to the MRI-PET cephalic molecular imaging coil in order to receive and process the signal coming from the MRI-PET cephalic molecular imaging coil, and the reconstruction and fusion workstation is connected to the signal processing assembly in order to receive and process the signal coming from the signal processing assembly and eventually to output molecular MR images. The signal processing assembly may include the MRI signal processing assembly which is connected to the MRI cephalic RF coil, and the PET signal processing assembly which is connected to the PET molecular signal acquisition assembly. The MRI signal processing assembly is designed for receiving and processing the signal coming from the MRI cephalic RF coil, and the PET signal processing assembly is designed for receiving and processing the signal coming from the PET molecular signal acquisition assembly. The reconstruction and fusion workstation is connected to the MRI signal processing assembly and the PET signal processing assembly respectively in order to receive and process the information coming from the latter two and to fuse and ultimately output molecular MR imagery.

Preferably, the signal processing assembly, the reconstruction and fusion workstation are external to the MRI-PET cephalic molecular imaging coil, becoming independent external structure.

The above MRI cephalic molecular imaging system of the present invention is capable of acquiring nuclear magnetic image and molecular imagery information at the same time, at the same location and during the same physiological period, thus generating precision molecular MR imagery.

### Beneficial Effects

At present, the molecular imaging MRI is the most advanced medical imaging technology in the world, and the combination of PET and MRI is an example, which includes separated type and integrated type. However, such equipment has huge space volume and expensive price. The technical scheme of the present invention provides for a nested structure and compact equipment, making it possible to be directly arranged in the access hole of the available MRI to acquire the MRI image and molecular image simultaneously at the same space. Therefore, it is able to synchronously acquire molecular MRI images on the same machine. Because images are generated under the same condition and state, the imaging information could be more precise and more objective for clinic studies and scientific research and the clinic results would be better and more precise. Additionally, the system or coil of the present invention is compatible directly with MRI equipment of the existing technology and its dimensions or sizes render it possible to be inserted in the existing MRI access holes. As a result, the equipment of the present invention is cost-effective and does not need extra civil works or machine rooms, greatly reducing the investment and increasing the utilization rate. Particularly, MRI now has been extensively applied in hospitals, so the equipment of the present invention can be directly used with the existing MRIs in hospitals, and therefore, it is greatly saved in manpower, material and financial resources.

### Description of Drawings

The present invention may be better understood by referring to the following drawings, in which:
Fig. 1 is a stereogram schematic view of the nested MRI-PET cephalic molecular imaging coil according to one embodiment of the present invention.
Fig. 2 is a front sectional view of the MRI-PET cephalic molecular imaging coil of Fig. 1.
Fig. 3 is an axially sectional view of the MRI-PET cephalic molecular imaging coil in Fig. 1, wherein the MRI coil is omitted and only the crystal array of the crystal gamma-ray detector is shown schematically.
Fig. 4 is a schematic view of the unit structure of the PET molecular signal acquisition assembly.
Fig. 5 is a schematic view of the nested MRI-PET cephalic molecular imaging system according to one embodiment of the present invention.
Fig. 6 is an actual example fusion image of the MRI-PET cephalic molecular imaging system.

### Example Embodiments

Understandably, the exemplary embodiments are given for illustrative purpose rather than limiting the invention.

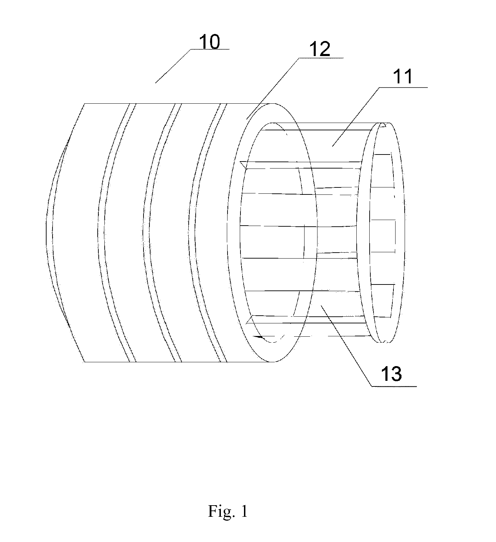

Fig. 1 shows a stereogram schematic view of the nested MRI-PET cephalic molecular imaging coil (10) according to one embodiment of the present invention, which includes the MRI cephalic RF coil (11) and the molecular signal acquisition assembly (12). Wherein, the MRI cephalic RF coil (11) may be the nuclear magnetic resonance cephalic RF coil of the existing technology, for emitting RF pulse and receiving MRI signal. The new type of nested MRI-PET cephalic molecular imaging coil of the present invention may be realized on the basis of the nuclear magnetic resonance cephalic RF coil of the existing technology. The molecular signal acquisition assembly (12) is located on the peripheral surface of the said MRI cephalic RF coil (11), being in tight contact with each other to form an integrated and nested structure. As shown in Fig. 1, the PET molecular signal acquisition assembly is attached to the peripheral surface of the MRI cephalic RF coil to form a ring structure. There may be provided some windows on the MRI cephalic RF coil (11), as shown in Fig. 1, and on a region of the MRI cephalic RF coil (11) not attached to the PET molecular signal acquisition assembly (12) such windows(13) are installed. The windows (13) can be multiple. Windows (13) help reduce the patient's claustrophobic sense when the device is surrounding the patient's head.

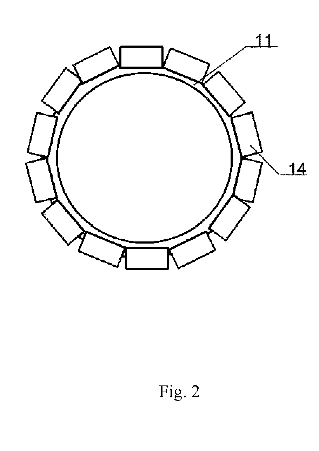

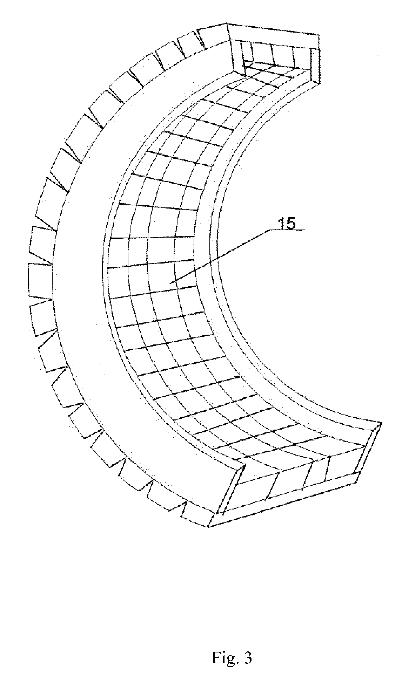

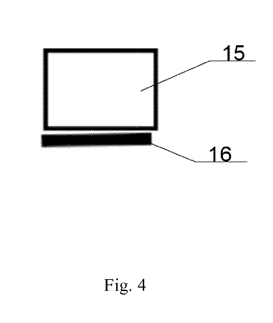

Fig. 2 is a front sectional view of the MRI-PET cephalic molecular imaging coil of Fig. 1. Fig. 3 is an axially sectional view of the MRI-PET cephalic molecular imaging coil in Fig. 1, wherein the MRI coil is omitted and only the crystal array of the crystal gamma-ray detector is shown schematically. Fig. 4 is a schematic view of the unit structure of the PET molecular signal acquisition assembly.

Reference to Fig. 2, 3 and 4, the PET molecular signal acquisition assembly (12) is located on the peripheral surface of the MRI cephalic RF coil (11), forming aring structure. The PET molecular signal acquisition assembly (12) includes multiple unit structures (14), each unit structure (14) is composed of a crystal gamma-ray detector unit (15) and a photoelectric conversion unit (16). Multiple unit structures are arrayed on the peripheral surface of the MRI cephalic RF coil to constitute the PET molecular signal acquisition assembly. In Fig. 3, for example, multiple unit structures of the PET molecular signal acquisition assembly attached to the MRI coil peripheral surface are arrayed to constitute the PET molecular signal acquisition assembly. The Fig. 3 only shows the crystal array formed by multiple gamma ray crystal detector units (15). In Fig. 4, an exploded view shows a combination of a crystal gamma-ray detector unit (15) and a photoelectric conversion structure unit (16). In fact, two of them are in tight contact with each other, for example, they may be combined together in a seamless manner.

More particularly, multiple crystal gamma-ray detector units (15) are attached to the peripheral surface of the MRI cephalic RF coil (11), thus forming a ring shape, and are used to convert gamma rays released during examination into visible lights. Multiple photoelectric conversion structure units (16) are attached to the surface of corresponding crystal gamma-ray detector units (15) to convert light signals into electric signals for sending out; then, electric signals are sent to the signal processing device for signal integration and the final fusion-images are formed by the image work station.

The crystal gamma-ray detector may be selected from BGO, LYSO, LSO, or LBS gamma-ray detection crystals and therefore the sensitivity and resolution are high. The photoelectric conversion structure may be semi-conductor structure, for converting light signals into electric signals. For instance, the semi-conductor structure being made of avalanche photodiodes may effectively avoid the interference of the magnetic field with the gamma ray imaging to realize more precise image.

Additionally, the PET molecular signal acquisition assembly (12) is directly on the MRI coil (11) so as to form a nested structure and thus the overall volume of the entire molecular imaging coil (10) is smaller; and two of them are overlapped on space to realize integration. Therefore, the molecular imaging coil of the present invention is compatible in dimension and size with the nuclear magnetic imaging equipment of the existing technology and the molecular imagery guarantees consistency of physiological and biochemical information in spatial location and temporal synchronization.

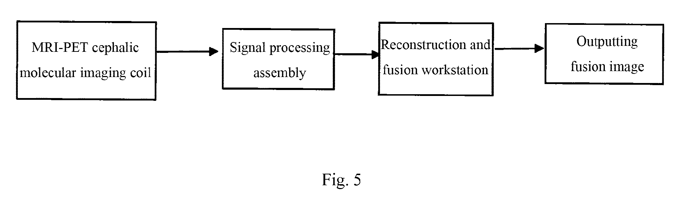

Fig. 5 is a schematic view of the nested MRI-PET cephalic molecular imaging system according to one embodiment of the present invention. As shown in this figure, this system includes MRI-PET cephalic molecular imaging coil, signal processing assembly, and reconstruction and fusion workstation, wherein the signal processing assembly is connected to the MRI-PET cephalic molecular imaging coil in order to receive and process the signal coming from the MRI-PET cephalic molecular imaging coil, the reconstruction and fusion workstation is connected to the signal processing assembly in order to receive and process the signal coming from the signal processing assembly and eventually to output molecular MR fusion-images.

The signal processing assembly may include the MRI signal processing assembly which is connected to the MRI cephalic RF coil, and the PET signal processing assembly which is connected to the PET molecular signal acquisition assembly. The MRI signal processing assembly is designed for receiving and processing the signal coming from the MRI cephalic RF coil, and the PET signal processing assembly is designed for receiving and processing the signal coming from the PET molecular signal acquisition assembly. The reconstruction and fusion workstation is connected to the MRI signal processing assembly and the PET signal processing assembly respectively in order to receive and process the information coming from the latter two and to fuse and ultimately output molecular MR imagery.

The signal processing assembly and the reconstruction and fusion workstation are external to the MRI-PET cephalic molecular imaging coil, becoming independent external structure. This arrangement is good to flexible combination of equipment and does not influence the structure or performance of the MRI-PET cephalic molecular imaging coil.

Prior to operation, radioactive isotope contrast agent may be injected into the patient. After waiting for a period of time (depending on what kind of tracer is used), the patient is introduced into the equipment for examination. As the conventional coil, when used, the coil is surrounded around the patient's head and is wired to be connected to the MRI and molecular imaging cabinet and is then introduced into MRI's patient hole for simultaneous nuclear medicine and nuclear image acquisition.

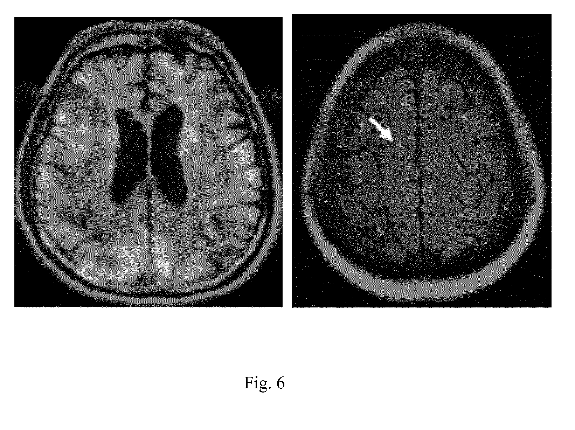

This integrated and nested design results in compact system equipment, which may be put directly into the MRI access hole for simultaneous MRI image and molecular image acquisition, and therefore it ensures consistency in spatial location, temporal simultaneity, and same physiological and biochemical information, thus generating the most precise fused images of magnetic resonance and molecular imagery. As shown on Fig. 6, the MRI-PET cephalic molecular imaging system of the present invention is capable of precision image fusion and the spot indicated by an arrow is the lesion spot. This fusion image ensures the consistency in spatial location, temporal simultaneity, and same physiological and biochemical information, thus more useful to clinic and scientific research.

Finally, it is worth noting that obviously the above example embodiments are furnished for the purpose of illustrating the present invention and are by no means any limitation to the ways of embodiment. Modifications or variations may be effected to the above embodiments by common technical personnel of the field to which the present invention is related. It is neither necessary nor possible here to exhaust all the embodiments. Apparent modifications or alterations derived therefrom are still in the protected scope of the present invention.

## Claims

1. An MRI-PET cephalic molecular imaging coil, including MRI cephalic RF coil and PET molecular signal acquisition assembly, wherein the said PET molecular signal acquisition assembly is located on the peripheral surface of the said MRI cephalic RF coil, hence being an integrated and nested structure, the said PET molecular signal acquisition assembly includes a crystal gamma-ray detector and a photoelectric conversion structure, the said photoelectric conversion structure is semi-conductors.

2. An MRI-PET cephalic molecular imaging coil according to Claim 1, wherein the said PET molecular signal acquisition assembly is attached to the peripheral surface of the said MRI cephalic RF coil so as to form a ring shape.

3. An MRI-PET cephalic molecular imaging coil according to Claim 2, wherein the said PET molecular signal acquisition assembly is attached to the peripheral surface of the said MRI cephalic RF coil and the regions of the said MRI cephalic RF coil not attached to the said PET molecular signal acquisition assembly are provided with windows.

4. An MRI-PET cephalic molecular imaging coil according to any of the above claim, wherein the said PET molecular signal acquisition assembly includes multiple unit structures, each unit structure is composed of a crystal gamma-ray detector unit and a photoelectric conversion structure unit.

5. An MRI-PET cephalic molecular imaging coil according to any of the above claims, wherein the said crystal gamma-ray detector is attached to the peripheral surface of the said MRI cephalic RF coil and the said photoelectric conversion structure is attached to the peripheral surface of the said crystal gamma-ray detector.

6. An MRI-PET cephalic molecular imaging coil according to any of the above claims, wherein the said crystal gamma-ray detector is selected from BGO, LYSO, LSO, or LBS gamma-ray detection crystals.

7. An MRI-PET cephalic molecular imaging system, includes the said MRI-PET cephalic molecular imaging coil of any of Claims 1 to 6, signal processing assembly, and reconstruction and fusion workstation,
wherein the said signal processing assembly is connected to the said MRI-PET cephalic molecular imaging coil in order to receive and process the signal coming from the said MRI-PET cephalic molecular imaging coil,
wherein the said reconstruction and fusion workstation is connected to the said signal processing assembly in order to receive and process the signal coming from the said signal processing assembly and eventually to output molecular MR fusion-images.

8. The system according to the above Claim 7, wherein the said signal processing assembly, the reconstruction and fusion workstation are external to the said MRI-PET cephalic molecular imaging coil, becoming the independent external structure.
